# FASCICULE DE BREVET EUROPEEN

(11) **EP 1 833 378 B1**
(45) Date de publication et mention de la délivrance du brevet: **23.10.2013**
(21) Numéro de dépôt: 05821575.7
(22) Date de dépôt: 21.12.2005
(51) Int. Cl.: A61B 17/02

(54) **ECARTEUR-RETENTEUR D'INTESTINS POUR CHIRURGIE COELIOSCOPIQUE**
-
RETRACTOR-RETAINER OF INTESTINES FOR CELIOSCOPIC SURGERY

(30) Priorité: 24.12.2004 FR 0413885
(43) Date de publication de la demande: 19.09.2007
(73) Titulaire: Surgical Perspective, 31000 Toulouse (FR); Université d'Aix-Marseille, 13284 Marseille Cedex 07 (FR); Assistance Publique Hôpitaux de Marseille, 13354 Marseille (FR)
(72) Inventeur: ALIMI, Yves, F-13012 Marseille (FR); MOURET, Frédéric, F-13005 Marseille (FR); RAMOS CLAMOTE, Joachim, F-13001 Marseille (FR)
(74) Mandataire: Decobert, Jean-Pascal
(86) Numéro de dépôt international: PCT/EP2005/057028
(87) Numéro de publication internationale: WO 2006/069947

(56) Documents cités:
- WO-A-03/096907
- DE-A1- 4 428 900
- FR-A- 2 805 731
- US-A- 5 803 902
- US-A1- 2001 016 754

## Description

La présente invention concerne un écarteur intestinal ou rétenteur d'intestins pour chirurgie coelioscopique. Il s'agit d'un instrument chirurgical invasif à usage temporaire destiné à être utilisé en chirurgie vidéo-assistée. Plus précisément, le dispositif a pour fonction de retenir les anses intestinales hors du champs de vision opératoire lors d'une intervention chirurgicale pratiquée dans l'abdomen particulièrement lors d'une intervention de restauration aortique abdominale, et de permettre l'intervention sans risque de contact avec les viscères écartés. Ce dispositif est par exemple utilisable en chirurgie digestive et vasculaire, afin de disséquer et réparer les organes ou vaisseaux à opérer. Cela permet de limiter le temps pendant lequel le patient est installé dans une position inconfortable pouvant provoquer des complications notamment au niveau respiratoire.

Plus de cent mille patients bénéficient chaque année d'une restauration aortique abdominale dans le monde, et les techniques chirurgicales conventionnelles sont associées à une morbidité et mortalité liées, en partie, à une large ouverture abdominale de 25 à 35 cm de longueur. Les techniques mini-invasives vidéo-assistées sont utilisées depuis plus de 20 ans en chirurgies gynécologiques et digestives, mais sont d'introduction plus récente en chirurgie aortique. Ceci est lié aux difficultés particulières que représente la dissection d'un organe profond et aux risques de saignement majeur en cas de malfaçon.

Un des premiers obstacles pour obtenir un accès à l'aorte est la présence de l'ensemble des anses intestinales qui doivent être écartées du champ opératoire pendant l'intervention. Les techniques laparoscopiques utilisées par les autres promoteurs de la chirurgie vasculaire ne sont pas nombreuses. Néanmoins deux grandes tendances se dégagent. La première consiste à mettre le patient sur le côté dans une position à forte inclinaison afin de faire "basculer" les intestins et donc laisser apparaître l'aorte. Malheureusement, l'accès à l'aorte ne peut plus se faire directement et la technique opératoire est plus difficile à acquérir. Les résultats obtenus avec cette technique sont engageants, mais il faut noter qu'ils sont, malgré une expérience importante, toujours légèrement moins bons que ceux obtenus par la chirurgie classique (taux de morbidité, de mortalité, de conversion,...). Ces résultats sont cependant décevants car le propre d'une nouvelle technique n'est pas de faire aussi bien mais d'apporter de réels avantages pour le patient.

Une autre approche est de chercher un accès direct à l'aorte sans mettre le patient dans une position inclinée inconfortable pendant plusieurs heures. Il est nécessaire, dans ce cas, d'utiliser un écarteur intestinal. Plusieurs systèmes ont été mis au point. L'un deux est constitué d'un écarteur composé d'un filet de forme carrée plié en deux et cousu de telle sorte qu'il forme une "pochette". A l'intérieur de cette poche, à deux extrémités du même côté, sont aménagées deux ouvertures qui permettent le passage de "râteaux" utilisés classiquement en chirurgie ouverte. Ces deux "râteaux" permettent de chaque côté de la poche de maintenir les intestins.

Cet écarteur présente plusieurs inconvénients. Il nécessite deux incisions, une pour chaque râteau. Sa mise en place n'est pas aisée car il faut introduire le filet et les deux râteaux par trois orifice différents puis seulement in situ insérer chacun des râteaux à l'intérieur du filet. Il est nécessaire de le maintenir à la main pour s'en servir car il n'y a pas de structure suffisamment rigide pour être mise en place de manière "définitive" pendant le temps de l'opération. Enfin, il ne permet pas de retenir correctement les intestins dans sa partie inférieure et ces derniers peuvent passer "par-dessous" et venir réencombrer le champ opératoire. En effet, en l'absence de structure rigide dans sa partie inférieure, celle-ci peut se déformer et laisser passer les intestins.

Le document FR 2805731 A1 présente lui aussi un écarteur doté d'un filet pour le positionnement et l'écartement des viscères intra-péritonéaux. Ce filet est fixable sur la paroi abdominale par deux points formant alors un dispositif à la façon d'un hamac. Une telle configuration n'offre aucune précision de placement et aucun élément de rigidité.

Un autre système proposé pour la chirurgie laparoscopique dans le domaine vasculaire, est constitué par un écarteur à cinq branches déjà utilisé en chirurgie digestive mais ce dernier présente de sérieux inconvénients. Il ne permet pas de maintenir la totalité des anses intestinales, et de ce fait doit être maintenu en permanence à la main pour libérer le champ opératoire. Le chirurgien, ou l'un de ses assistants, est, dans ce cas, privé de l'usage de l'une de ses mains.

Afin d'éviter les risques hémorragiques de ces techniques laparoscopiques réalisées à ventre fermé, il a été proposé une technique opératoire dite du "Hand-Port", qui permet la pénétration de la main non dominante du chirurgien dans l'abdomen du patient, par une incision de 7 à 8 cm, tout en maintenant par un système de manchette étanche, la création d'un pneumopéritoine à la pression constante de 14 mmHg. Cette main permet d'écarter les viscères et de comprimer un vaisseau en cas d'hémorragie brutale ; l'autre main du chirurgien réalise la dissection aortique et la confection des anastomoses aortiques, sous contrôle vidéo de la caméra maintenue par un assistant. Cette technique a été abandonnée pour plusieurs raisons. Tout d'abord, le chirurgien a une main occupée par l'écartement des viscères et même si ce système est plutôt rassurant pour les chirurgiens débutants cette technique, elle, ne va pas dans le sens de la chirurgie totalement laparoscopique.

L'écarteur intestinal dans le cas de la chirurgie vasculaire est destiné à être utilisé chez des patients fragiles et âgés (souvent, et de plus en plus, de 80 à 90 ans) beaucoup plus que chirurgie digestive (50 - 60 ans). De ce fait, les techniques utilisées dans les deux dernières chirurgies pour écarter les intestins sont plus risquées pour les patients. En effet, on utilise généralement la gravité pour écarter les intestins, on positionne le patient en décubitus latéral droit et en Trendelenbourg (tête vers le bas). En outre, l'aorte est un organe très profond au niveau de l'abdomen et de plus médiane, cela impose une déclivité extrême des positions pouvant entraîner les complications déjà citées plus des oedèmes cérébraux (travaux de Barbosa), d'où l'utilité d'un écarteur intestinal.
Des dispositifs écarteurs ont été proposés pour dégager le champ de vision à l'intérieur de la cavité abdominale durant une intervention laparoscopique.

Le document US-5.465.711 décrit un écarteur intestinal gonflable utilisable en chirurgie endoscopique. Cet écarteur gonflable permet de repousser les intestins afin de ménager un accès à des organes tels que colonne vertébrale, aorte, reins, etc. Dans certaines applications telles que les interventions aortiques abdominales, cet écarteur présente l'inconvénient de ne pas pouvoir être adapté in situ à l'anatomie du patient, par le chirurgien. Il a une forme prédéfinie et est gonflé de manière à obtenir une forme et un volume prédéterminés permettant de dégager un accès à l'organe malade.

Le document US-2003/0004473 décrit un dispositif rétenteur de viscères déformable constitué d'une enveloppe gonflable rectangulaire essentiellement plane dans laquelle sont logées des feuilles dont certaines au moins sont réalisées dans un matériau malléable. Un tel dispositif encombrant ne peut être utilisé qu'en chirurgie ouverte et non pas, bien entendu, en chirurgie mini invasive.

Le document US-5.318.586 décrit un dispositif utilisable en chirurgie laparoscopique. Ce dispositif peut servir à écarter les intestins ou autres organes. Il comprend un écarteur constitué par un embout gonflable logé avec une possibilité de coulissement dans un tube rigide destiné à être introduit dans l'abdomen, cet embout pouvant être poussé hors de l'extrémité distale du tube rigide et dilaté au moyen d'un gaz ou d'un fluide, ledit embout pouvant avoir différentes formes et tailles selon les interventions auxquelles est destiné le dispositif. L'embout gonflable possède, dans tous les cas, une forme prédéterminée et le tube d'introduction et de guidage est rigide, de sorte que le dispositif ne peut être adapté in situ à l'anatomie du patient.

Le document DE-A1-4428900 divulgue un dispositif chirurgical pour opérations laparoscopiques, comportant une paire de barres disposées parallèlement pour faire office de support à un élément d'appui fixé de manière amovible aux extrémités des barres. Cette structure est complexe et volumineuse.

Le document US 5 803 902 est un rétracteur chirurgical doté d'un manche et d'un dispositif déployable par un mécanisme commandable. Il est de faible amplitude de surface de rétraction.

L'examen des méthodes et dispositifs divulgués par l'état de la technique, permet de constater que le besoin d'avoir à disposition un dispositif qui soit facilement adaptable "in situ" à n'importe quelle approche chirurgicale, qui empêche tout déplacement des intestins une fois positionné et libère le chirurgien de toute contrainte lors d'une opération, n'a pas encore été satisfait.

L'invention propose un dispositif écarteur et rétenteur d'intestins utilisable en chirurgie laparoscopique apportant une solution au problème d'inadéquation à l'anatomie du patient rencontré avec les dispositifs de l'état de la technique, et dont la mise en place peut s'opérer dans un temps réduit.

Selon l'invention, cet objectif est atteint grâce à un dispositif comprenant :
- un mandrin directeur comportant une partie proximale constituée par un manche apte à être relié à une table d'opération, une partie distale de forme allongée appelée à être introduite dans la cavité abdominale d'un patient, lors d'une intervention laparoscopique ;
- un mandrin d'appui sous forme de gouttière allongée sur lequel et le long duquel est fixée une nappe de contention tel un filet replié selon un mode de pliage permettant son déploiement in situ, l'extrémité distale de ce mandrin d'appui et la partie distale du mandrin directeur étant pourvues de moyens complémentaires d'accouplement rapide ;
- et un instrument pour l'insertion du mandrin d'appui et du filet de contention dans la cavité abdominale, et la fixation dudit mandrin d'appui sur le mandrin directeur, cet instrument comportant un canon dont au moins la partie distale est creuse et dimensionnée pour recevoir ledit mandrin d'appui et le filet de contention relié à ce dernier.

Selon un mode d'exécution préféré, la partie distale du mandrin directeur est constituée par un guide tubulaire, dans lequel est logée une tige d'accouplement montée avec une aptitude de mouvement axial dans ledit guide tubulaire dont l'extrémité distale est munie d'un passage transversal, l'extrémité distale du mandrin d'appui comportant un ergot destiné à s'insérer dans ledit passage transversal et muni d'un cran ou entaille transversale dans laquelle peut être engagée l'extrémité distale de la tige d'accouplement, de sorte à fixer le mandrin d'appui au mandrin directeur.

Selon un mode d'exécution, la tige d'accouplement est soumise à l'action d'un ressort tendant à la repousser en position d'accouplement, de sorte que la fixation du mandrin d'appui au mandrin directeur s'opère automatiquement par simple enfoncement de l'ergot d'accouplement dudit mandrin d'appui dans le passage transversal dudit mandrin directeur.

Selon un autre mode d'exécution, le manche du mandrin directeur est équipé d'un mécanisme à commande par bouton-poussoir permettant d'obtenir, alternativement, par des pressions successives sur ce bouton-poussoir, soit l'introduction de l'extrémité distale de la tige d'accouplement dans l'entaille transversale de l'ergot d'accouplement du mandrin d'appui, soit la rétraction de ladite tige.

Le dispositif selon l'invention procure des avantages intéressants ; il permet notamment, lors d'une intervention de restauration aortique abdominale :
- une parfaite adaptation in situ, à l'anatomie du patient ;
- une fixation stable à la table d'opération : le maintien en position est assuré une fois pour toute, le chirurgien et les assistants ont les mains libres et n'ont pas à veiller au maintien du dégagement du champ opératoire ;
- une mise en place réalisable dans un temps relativement réduit pour ce type d'intervention, de l'ordre de 15 à 20 minutes ;
- un abord direct de l'aorte abdominale, en évitant donc les larges dissections rétropéritonéales ;
- la constitution d'une barrière infranchissable pour les anses intestinales, durant toute la durée de l'intervention ;
- le maintien du patient en décubitus dorsal (à plat sur le dos), en évitant les positions prolongées de Trendelenbourg à 25° (tête en bas) et en décubitus dorsal droit entre 25 et 60° (patient couché sur le côté droit), qui peuvent avoir des effets délétères chez des patients âgés avec une fonction cardio-respiratoire déficiente (des atélectasies des apex pulmonaires, des oedèmes ophtalmiques et cérébraux ont été constatés avec certaines méthodes actuellement appliquées) ;
- une réduction de la pression du pneumopéritoine (gaz insufflé dans le ventre) de 14 à 8 mm de Hg, avec une diminution de retentissement digestif et rénal (ischémie capillaire sur le territoire splanchnique et oligo-anurie).

Les buts, caractéristiques et avantages ci-dessus, et d'autres encore, ressortiront mieux de la description qui suit et des dessins annexés dans lesquels :

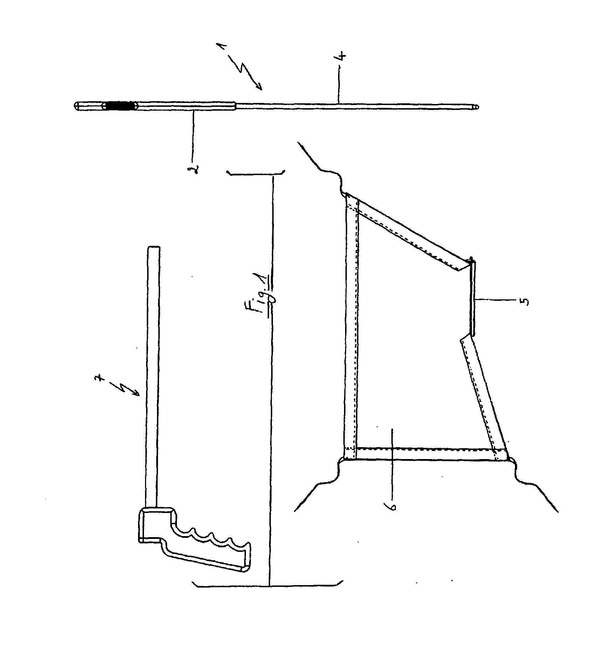

La figure 1 est une vue illustrant, séparément, les parties composantes du dispositif écarteur-rétenteur d'intestins selon l'invention.

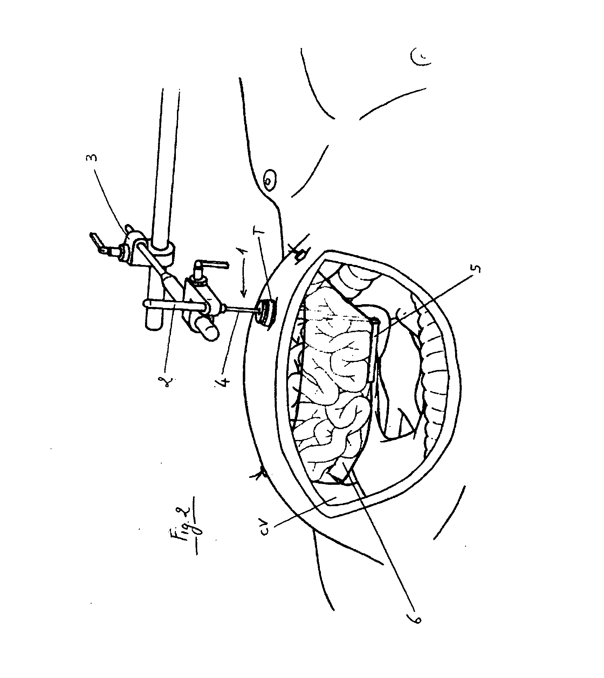

La figure 2, est une vue en perspective montrant l'écarteur-rétenteur selon l'invention dont les parties invasives sont logées dans la cavité abdominale d'un patient et dont la partie extérieure est raccordée à un dispositif de fixation à une table d'opération partiellement représenté.

La figure 3 est une vue en coupe axiale d'un premier exemple de réalisation du mandrin directeur.

La figure 4A est une vue en perspective et en plongée avant du mandrin d'appui.

La figure 4B est une vue en perspective et en plongée arrière de ce mandrin d'appui.

La figure 5A est une vue en coupe longitudinale dudit mandrin.

La figure 5B est une vue de face de la tête d'accouplement de ce dernier.

La figure 5C est une vue en coupe selon la ligne 5C-5C de la figure 5A.

La figure 6A est une vue en perspective de la tête d'accouplement du mandrin d'appui.

La figure 6B est une vue de dessus de la figure 6A.

La figure 7 est une vue de face d'un filet de contention à l'état déployé.

Les figures 8 et 9 sont des vues illustrant deux étapes de réalisation d'une bordure renforcée du filet.

Les figures 10A à 10F illustrent les étapes d'un mode de pliage du filet de contention de sorte à autoriser son introduction dans le canon de l'instrument d'insertion.

La figure 11A est une vue partielle et les figures 11B et 11C sont des vues en perspective illustrant le positionnement des aiguilles de fixation dans le filet de contention plié.

La figure 12 illustre un autre mode de positionnement des aiguilles de fixation, sur le filet représenté à l'état déployé.

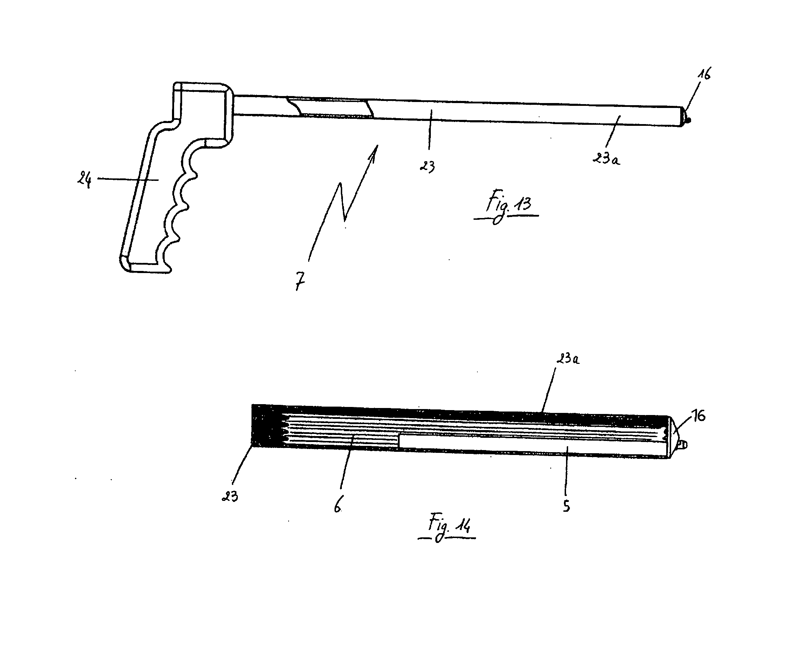

La figure 13 est une vue en élévation, avec coupe partielle, de l'instrument d'insertion du mandrin d'appui et du filet de contention.

La figure 14 est une vue en coupe longitudinale et à plus grande échelle de la partie distale du tube d'insertion dans laquelle sont positionnés le mandrin d'appui et le filet de contention.

La figure 15 est une vue en perspective montrant l'insertion du mandrin directeur et du mandrin d'appui supportant le filet, dans la cavité abdominale.

Les figures 16A, 16B, 16C, illustrent, par des vues en coupe axiale, le mode de clipsage du mandrin d'appui sur le mandrin directeur.

La figure 17 est une vue en coupe axiale illustrant la séparation du mandrin d'appui et du mandrin directeur, en fin d'intervention.

Les figures 18A à 18D illustrent un mode de fixation du mandrin d'appui sur un mandrin directeur, au moyen d'un agencement et d'un fonctionnement différents de ce dernier.

On se reporte auxdits dessins pour décrire des modes de réalisation intéressants, mais nullement limitatifs, de l'écarteur-rétenteur d'intestins selon l'invention.

Cet écarteur-rétenteur comprend :
- un mandrin directeur 1 comportant une partie proximale 2 constituée par un manche conformé pour permettre sa fixation, dans la position souhaitée, à une table d'opération, au moyen d'un dispositif de fixation 3 qui peut être d'un type connu en soi, et une partie distale 4 de forme allongée appelée à être introduite, au moyen d'un trocart T dans la cavité abdominale C d'un patient lors d'une intervention laparoscopique;
- un mandrin d'appui 5 de forme allongée ; et
- une nappe tel un filet de contention 6.

Le dispositif selon l'invention concerne encore un instrument 7 pour l'insertion du mandrin d'appui et du filet de contention dans la cavité abdominale. Cet instrument comporte une poignée et une tige dont au moins la partie distale est creuse pour le logement dudit mandrin d'appui et dudit filet de contention, avant introduction dans la cavité abdominale.

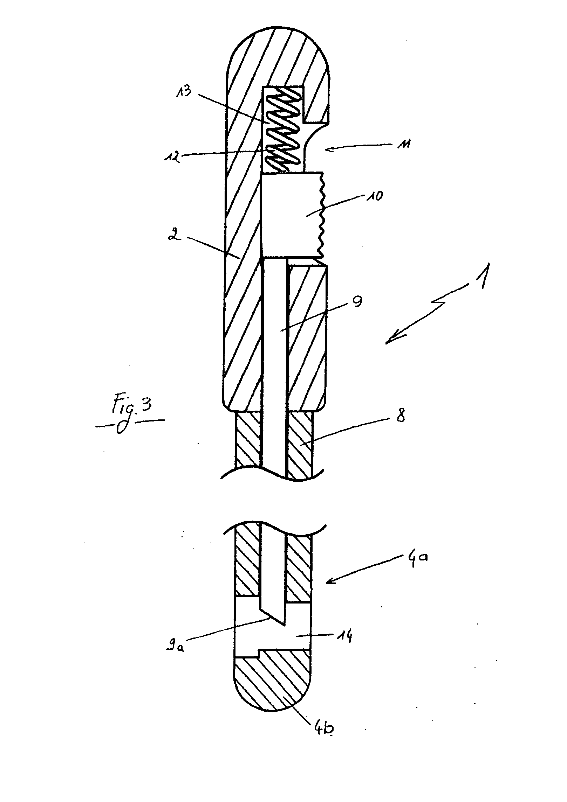

Le mandrin directeur 1 comprend, selon un premier mode d'exécution plus particulièrement illustré à la figure 3, un manche 2 et un tube 8 solidaire du manche 2 et disposé, coaxialement, dans le prolongement de ce dernier.

La partie terminale 4a du tube 8 constituée par la partie distale 4 du mandrin directeur 1 est agencée pour former l'élément femelle auquel se fixe l'extrémité distale du mandrin d'appui 5. Dans le tube 8 et dans le manche 2, est montée, avec une aptitude de mouvement axial, une tige d'accouplement 9 dont l'extrémité proximale est solidaire d'un bouton de manoeuvre 10 accessible à travers une ouverture 11 ménagée latéralement dans le manche 2. Un ressort hélicoïdal 12 calé, par l'intermédiaire de ses extrémités opposées, d'une part, contre le fond du passage axial borgne 13 ménagé dans le manche 2, et, d'autre part, contre la face supérieure du bouton de manoeuvre 10, tend à rappeler l'ensemble mobile bouton 10-tige d'accouplement 9 en position active de clipsage. La partie distale 4a du tube 8 est munie d'un passage transversal avec chambrage 14, d'axe perpendiculaire à l'axe de la tige d'accouplement 9 et en travers duquel peut se déplacer l'extrémité distale pourvue d'un biseau 9a de cette dernière.

L'extrémité 4b de la partie distale 4 du mandrin directeur 1 présente une forme arrondie, par exemple sphérique ou en forme de calotte sphérique, afin d'éviter tout risque de blessure des tissus ou organes lors de la manipulation et de la mise en place dudit mandrin directeur.

A titre indicatif, le corps tubulaire 8 du mandrin directeur peut avoir une longueur au moins égale à 200 mm et un diamètre externe maximum de 5 mm, pour pouvoir être passé à travers un trocart de 5. Le manche 2 peut avoir une longueur supérieure à 150 mm et un diamètre supérieur à 5 mm afin de pouvoir être serré dans un système de fixation connu en soi à la table d'opération et contenir le bouton 10, le ressort 12 et une partie de la tige 9.

Le mandrin directeur 1 tel que décrit ci-dessus peut être réalisé en métal inoxydable, ou en matière plastique rigide, ou en une combinaison des deux.

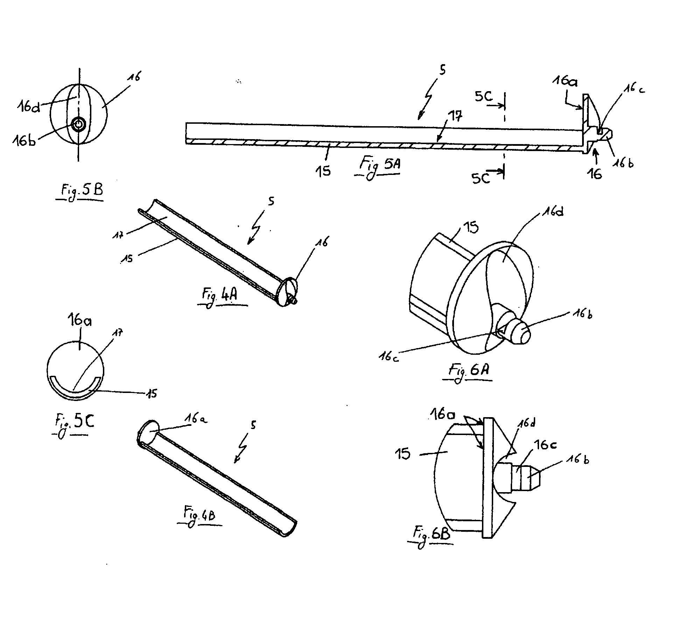

Le mandrin d'appui 5 selon l'exemple de réalisation illustré aux figures 4 à 6, comprend une gouttière 15 dont la longueur peut être comprise entre 80 et 100 mm et une tête de clipsage 16 constituant la partie distale de cette gouttière. Cette dernière présente, de préférence, une section en arc de cercle et délimite ainsi une gorge longitudinale 17 pour la réception du filet de contention 6 à l'état plié.

La face arrière de la tête d'accouplement 16 remplit la fonction d'un arrêtoir 16a de forme circulaire ou autre présentant un diamètre ou des dimensions supérieures à l'alésage du canon de l'instrument d'insertion dans lequel doit être engagé le mandrin d'appui en vue de son introduction dans la cavité abdominale, par exemple un diamètre de 12 mm qui est le diamètre maximum pour les interventions envisagées.

La face avant de cet arrêtoir est munie d'un ergot d'accouplement 16b, par exemple de forme tronconique, pourvue d'une entaille transversale ou cran 16c avec chambrage et constituant l'extrémité distale du mandrin d'appui. D'autre part, la face avant de la tête d'accouplement 16 comporte une cavité diamétrale 16d de forme incurvée, cette cavité étant conformée pour épouser la forme cylindrique de la paroi latérale de la partie distale 4 du mandrin directeur 1. Cet agencement permet de définir une et une seule bonne position du mandrin d'appui 5 par rapport au mandrin directeur 1, ce qui facilite grandement la manipulation lors de l'arrimage dudit mandrin d'appui audit mandrin directeur.

Le filet de contention 6 constituant la nappe de contention dans l'exemple ici décrit est rattaché à la gouttière 15 dans laquelle il est logé à l'état plié en vue de son introduction dans la cavité abdominale.

La liaison entre le mandrin d'appui 5 et le filet de contention 6 dépend, entre autre, des matériaux dans lesquels sont fabriqués ces deux éléments. Suivant la nature de ces matériaux, la liaison peut être faite :
- par couture : le mandrin est percé de trous répartis sur la longueur de la gouttière ; la couture est réalisée lors de la fabrication grâce à un fil adapté passant dans les trous du mandrin d'appui et dans les mailles du filet ;
- par collage ;
- par thermosoudage ;
- ou par d'autres procédés.

Le filet 6 est confectionné à l'aide d'un fil extrêmement fin et élastique lui-même réalisé dans un matériau présentant les qualités requises. D'autres structures, notamment non tissées, étanches ou non, peuvent remplir la fonction de nappe de façon similaire à un filet.

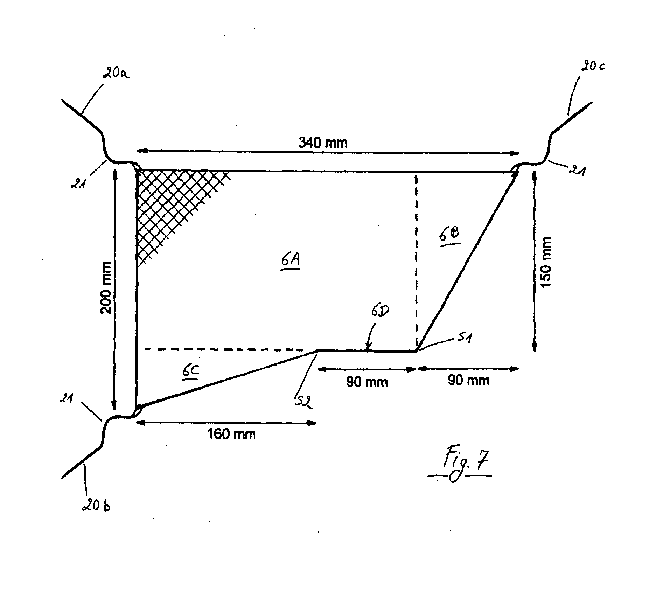

Il peut avantageusement avoir la forme illustrée à la figure 7 et les dimensions indiquées sur cette figure. Ce mode d'exécution présente une forme constituée par : - une grande surface rectangulaire 6A, - une première surface triangulaire 6B constituée par un triangle rectangle rattaché par son grand côté de l'angle droit à l'un des petits côtés du rectangle, et - une deuxième surface triangulaire 6C constituée par un triangle rectangle rattaché par son grand côté de l'angle droit à une portion de l'un des grands côtés de ladite surface rectangulaire. Le petit côté de l'angle droit du premier triangle 6B se trouve disposé dans le prolongement de l'un des grands côtés du rectangle 6A tandis que le petit côté de l'angle droit du deuxième triangle 6C se trouve disposé dans le prolongement du petit côté du rectangle 6A qui forme un angle droit avec le grand côté dudit rectangle prolongé par le petit côté de l'angle droit dudit premier triangle 6B.

A titre d'exemple, la partie principale rectangulaire 6A peut avoir une longueur de 250 mm et une largeur de 150 mm, les côtés de l'angle droit du triangle 6B peuvent avoir des longueurs de 150 mm et 90 mm, respectivement, et les côtés de l'angle droit du triangle 6C peuvent présenter des longueurs de 160 mm et 50 mm, respectivement.

Le filet de contention 6 ainsi conformé est fixé au mandrin d'appui 5 par l'intermédiaire de sa bordure 6D délimitée par les sommets S1 et S2 des surfaces triangulaires 6B et 6C, constitués par les angles formés par leur hypoténuse et le grand côté de leur angle droit.

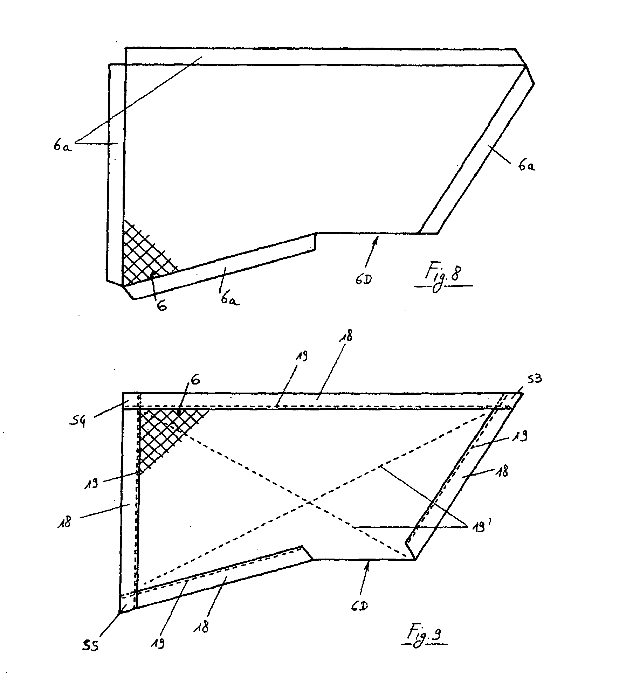

Le bord libre du filet de contention 6, notamment lorsque celui-ci présente un degré d'élasticité, peut être avantageusement renforcé. De préférence, le bord du filet peut être renforcé par une bordure 18 qui peut être formée par de simples revers cousus par des fils non élastiques 19 (figure 9). dans ce cas, le filet est initialement découpé de manière à permettre la réalisation de revers 6a sur les bords du filet (figure 8).

La double utilité de ce renfort est à la fois d'augmenter la résistance du filet de contention :
- d'une part, le long de ses bords qui présentent ainsi deux épaisseurs de tissus, et, également,
- d'autre part, aux angles ou sommets S3, S4 et S5 du filet destinés à être attachés à la paroi abdominale interne par l'intermédiaire d'aiguilles et qui présentent, de la sorte, trois épaisseurs de tissus à ces emplacements.

On observe que le matériau utilisé pour constituer le filet est extrêmement fin et élastique. Sa finesse est un atout prépondérant car elle solutionne le problème de retrait final : en fin d'opération le filet est complètement déplié mais il est tellement fin qu'il peut être retiré sans pliage quelconque, à travers un trocart. Par contre, son élasticité est un désavantage car une certaine rigidité est nécessaire pour bien maintenir les intestins. C'est pourquoi les fils utilisés pour coudre les revers offrent une solution à ce désavantage : eux sont non élastiques et rigidifient le filet en ses bords. Pour rigidifier la partie centrale, on pourra même rajouter des fils 19' suivant les diagonales du filet (figure 9).

Le filet de contention 6 est bien évidemment plié pour pouvoir être inséré dans le canon de l'ustensile d'insertion, avec le mandrin d'appui 5, via un trocart. Toute configuration de pliage entre dans le cadre de l'invention, en accordéon ou en enroulement notamment

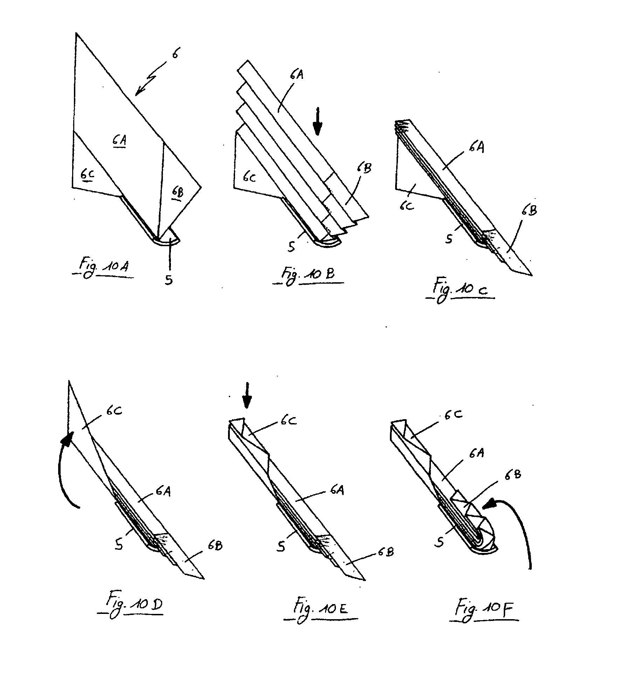

Les figures 10A à 10F sont des vues à caractère schématique illustrant un mode de pliage avantageux du filet.

La figure 10A montre le filet déployé et fixé par son bord 6D dans la gouttière du mandrin d'appui.

Les figures 10B et 10C représentent deux étapes du pliage en accordéon des parties 6A et 6B du filet.

Sur la figure 10D, on voit que la partie 6C est ramenée au-dessus de la partie 6A pliée.

La partie 6C est à son tour pliée en accordéon et ce pliage se trouve ainsi par dessus le pliage de la partie 6A, comme le montre la figure 10E.

La figure 10F montre le pliage de la partie 6B à son tour ramenée par dessus celui de la partie 6A.

Cette forme repliée peut ensuite être logée dans la gouttière du mandrin d'appui et insérée, avec celui-ci, à l'intérieur du canon de l'instrument d'insertion pour être délivrée.

Comme indiqué précédemment, les sommets S3, S4, S5 du filet 6 sont destinés à être attachés à la paroi abdominale interne, au moyen d'un lien et d'aiguilles 20a, 20b, 20c.

Selon une disposition caractéristique de l'invention, ces aiguilles sont rattachées d'origine aux sommets S3, S4, S5 du filet 6, ce qui signifie que, lors de la fabrication du filet de contention, chacun de ses angles ou sommets S3, S4, S5, doit être muni d'un fil 21 et d'une aiguille 20, comme le montre la figure 7.

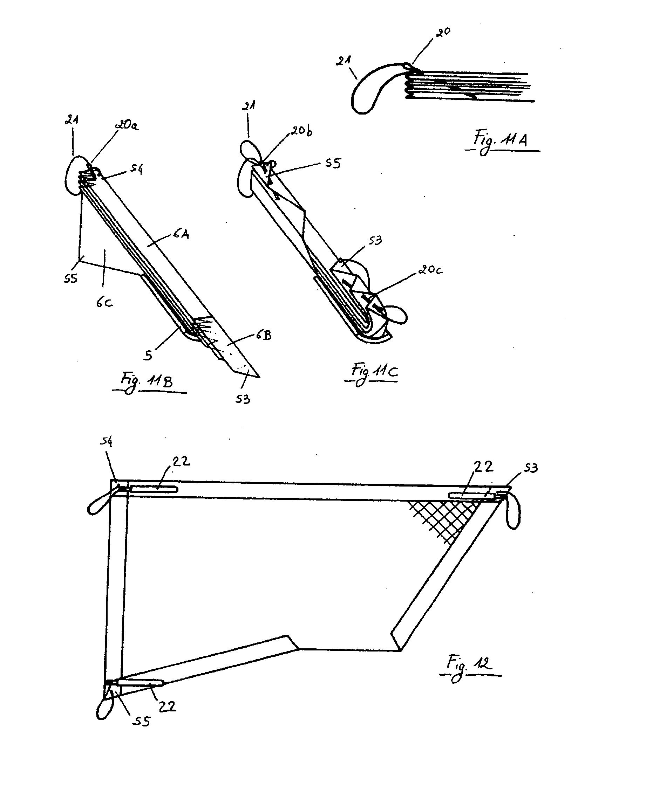

Selon un premier mode de réalisation illustré aux figures 11A à 11C, les aiguilles sont simplement piquées dans les épaisseurs du pliage, ce qui présente l'avantage de favoriser le maintien du pliage en accordéon lequel a naturellement tendance à se déplier.

L'aiguille 20a reliée au sommet S4 (la dernière à être utilisée par le chirurgien lors de la mise en place du filet) est piquée dans le pliage de la partie 6A (figure 11B) au stade du processus de pliage illustré à la figure 10C.

L'aiguille 20b reliée au sommet S5, est piquée dans le pliage des parties 6C et 6A (figure 11C) au stade du processus de pliage illustré à la figure 10E, tandis que l'aiguille 20c reliée au sommet S3 du filet, est piquée dans le pliage des parties 6B et 6A (figure 11C) au stade du processus de pliage montré à la figure 10F.

Selon un autre mode d'exécution illustré à la figure 12, le filet de contention 6 est muni, à proximité de chacun des angles ou sommets S3, S4, S5, d'un petit fourreau 22 dans lequel peut être logée l'aiguille 20 rattachée par un fil 21 au sommet S3, S4 ou S5 respectif. De la sorte, il n'existe plus de risque que les pointes des aiguilles puissent traverser la totalité du filet replié et blesser les tissus environnants. Par contre les aiguilles logées dans les fourreaux ne peuvent pas être utilisées pour maintenir le pliage, puisqu'elles ne sont pas traversantes.

L'instrument d'insertion 7 qui complète le dispositif selon l'invention peut être constitué très simplement par une tige 23 dont au moins la partie distale 23a est creuse. De préférence, cette tige est constituée par un tube cylindrique dont l'extrémité proximale est rattachée à une poignée 24.

L'ensemble poignée 24-tube 23 reproduit approximativement la forme d'un "pistolet" ; pour cette raison, il est désigné par ce mot dans la description qui suit, tandis que ledit tube est appelé "canon".

Le mandrin d'appui 5 et le filet de contention replié 6 rattaché à celui-ci sont engagés dans la partie distale 23a du canon 23. Dans cette position, l'arrêtoir 16a se trouve en butée contre l'extrémité distale du canon 23, de sorte que la tête d'accouplement 16 du mandrin d'appui occupe une position émergente par rapport à ladite extrémité. Cette disposition autorise une fixation automatique, par clipsage, de l'extrémité distale du mandrin d'appui sur la partie distale du mandrin directeur, comme cela est décrit dans la suite du présent exposé.

Selon le mode d'exécution illustré, le mandrin d'appui et le filet sont maintenus à l'intérieur du canon par les seuls frottements liés à leur encombrement. Le pistolet peut aussi être équipé d'un système dédié au largage du mandrin d'appui.

Le pistolet 7 n'est pas réutilisable. Le canon 23 peut contenir un système qui empêche sa réutilisation. Par exemple, le canon peut être équipé d'un système à double fond qui est libéré lors de l'extraction du filet et qui ne peut plus revenir à sa position initiale.

Le pistolet n'a pour fonction que la pose du filet et du mandrin d'appui. Le retrait de ces derniers, à la fin de l'intervention chirurgicale, se fait directement par le chirurgien à travers les trocarts. Celui-ci, après avoir coupé les fils de maintien à la paroi et libéré le mandrin d'appui du mandrin directeur, attrape le filet dans une zone proche du mandrin d'appui et retire le tout à travers le trocart à l'aide d'une pince couramment utilisée en chirurgie vasculaire.

On décrit ci-après le mode opératoire utilisant l'écarteur-rétenteur d'intestins selon l'invention.

En prenant pour exemple une intervention de restauration aortique abdominale, en chirurgie laparoscopique ou coelioscopique, le patient est placé sur la table d'opération en décubitus latéral droit et Trendelenbourg à 25°. Dans cette position, la pesanteur amène naturellement les intestins vers la paroi abdominale droite, ce qui dégage le péritoine et la zone de dissection.

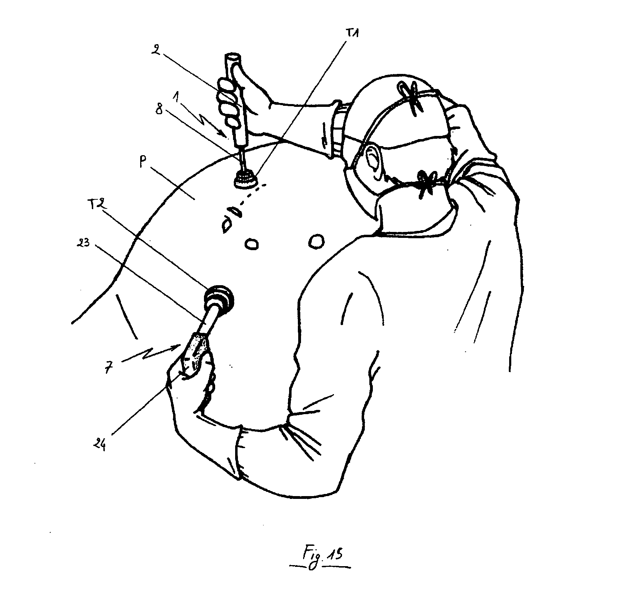

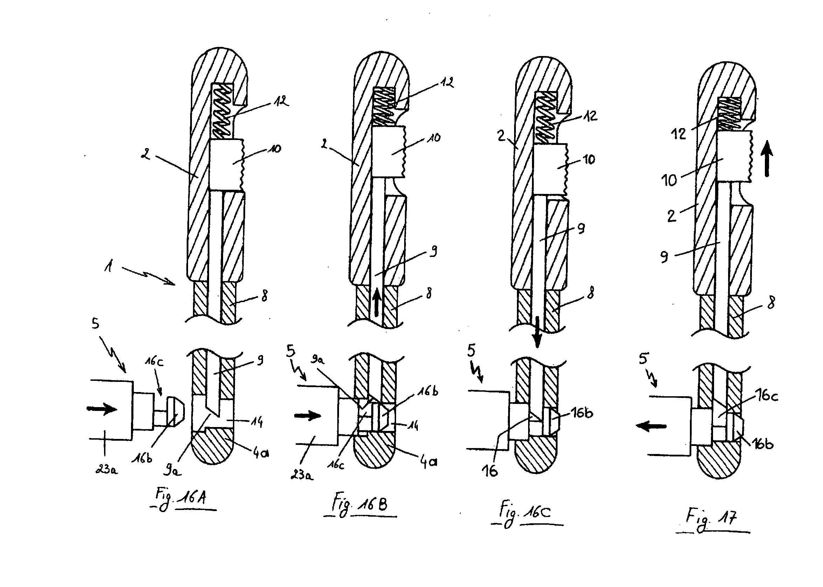

Par l'intermédiaire d'un premier trocart T1 de taille 5, le mandrin directeur 1 est enfoncé dans la paroi abdominale P et il est fixé, à l'extérieur de ladite paroi, par l'intermédiaire de son manche 2, à la table d'opération, au moyen d'un dispositif d'attache 3 qui peut être d'un type connu en soi. Par l'intermédiaire d'un autre trocart T2, de taille maximale 12, le mandrin d'appui 5 et le filet 6 sont introduits dans la cavité abdominale C, au moyen du pistolet 7 (figure 15). Dans cette phase d'introduction, le chirurgien manipule donc ce mandrin depuis l'extérieur de la cavité abdominale C, par l'intermédiaire du pistolet 7. Il amènera ensuite l'extrémité distale du mandrin d'appui vers la partie distale du mandrin directeur (figure 16A).

Lors de son introduction dans le passage transversal 14 de la partie distale du mandrin directeur 1, le tenon d'accouplement tronconique 16b glisse sur le biseau 9a de la tige de blocage 9, en entraînant un déplacement vers le haut de ladite tige et du bouton 10, à l'encontre de l'action antagoniste du ressort 12 (figure 16B).

En poursuivant le mouvement d'introduction du tenon d'accouplement 16b, la tige mobile de blocage 9 retombe dans l'entaille transversale 6c située en arrière dudit tenon, sous la pression exercée par le ressort 12 (figure 16C). Le mandrin d'appui se trouve ainsi accouplé à la partie distale du mandrin directeur.

Il est ensuite possible de retirer le pistolet 7, en laissant en place le mandrin d'appui 5 attaché au mandrin directeur 1.

Le retrait du pistolet permet de dévoiler le filet plié sur le mandrin d'appui.

Les aiguilles et le fil qui vont permettre d'attacher le filet à la paroi abdominale sont déjà solidaires du filet. Le chirurgien se saisit de la première aiguille et l'amène vers l'endroit de la paroi abdominale où il veut la faire passer, ce qui déploie en partie le filet. Puis de même pour les deux autres aiguilles.

Comme indiqué précédemment, les aiguilles 20a, 20b, 20c rattachées aux sommets S4, S5, S3, respectivement, doivent apparaître dans un ordre déterminé pour simplifier la mise en place du filet, après le retrait du pistolet.

L'aiguille 20c rattachée au sommet S3 doit apparaître en premier, le chirurgien s'en saisit et lui fait traverser la paroi abdominale en I, ce qui entraîne le déploiement partiel du filet. Puis l'aiguille 20b reliée au sommet S5 est amenée au point distal de la racine du mésentère, en II. L'aiguille 20a rattachée au sommet S4 est amenée en III.

Le filet se trouve alors totalement déployé et fixé et l'écarteur-rétenteur d'intestins selon l'invention est installé.

La partie 6B du filet retient le colon transverse.

La partie 6A dudit filet retient la face F.

La partie 6C de ce dernier retient les intestins le long du mésentère.

Pour obtenir la séparation du mandrin d'appui et du mandrin directeur, à la fin de l'intervention chirurgicale, il suffit de soulever le bouton 10 relié à la tige de clipsage 9, au niveau du manche 2, pour libérer ledit mandrin d'appui et permettre son retrait (figure 17).

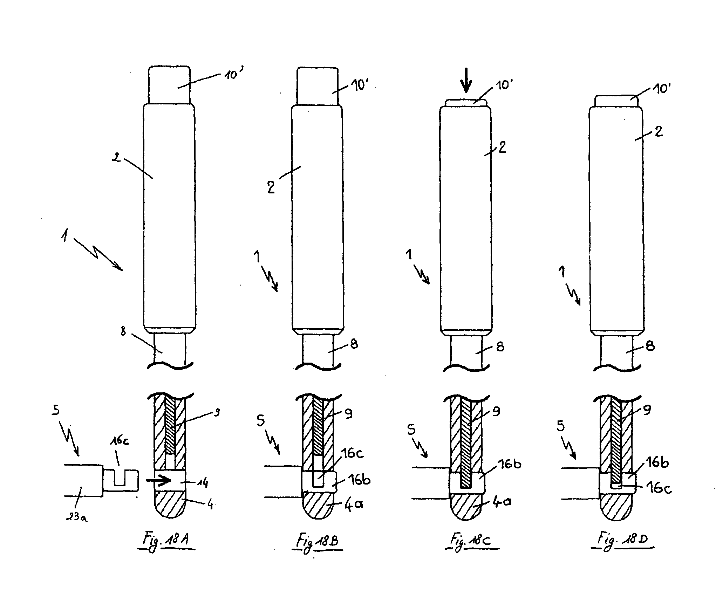

Les figures 18A à 18D illustrent un deuxième exemple de réalisation du mandrin-directeur et du mode de fixation du mandrin d'appui 5 sur celui-ci.

Sur ces figures, les parties composantes du mandrin directeur 1 et du mandrin d'appui 5 identiques ou quasi identiques aux parties composantes du mode d'exécution précédemment décrit, sont désignés par les mêmes références.

Les déplacements axiaux de la tige d'accouplement 9 dans le tube 8 constituant la partie distale 4 du mandrin-directeur sont commandés par un bouton-poussoir 25 émergeant à l'extrémité supérieure du manche 2 et permettant d'actionner un mécanisme (non représenté) logé dans ledit manche, pour obtenir, alternativement, par des pressions successives sur ce bouton-poussoir, soit l'introduction de l'extrémité de la tige d'accouplement 9 dans le passage transversal 14, soit la rétraction de ladite extrémité dans le tube 8. De tels mécanismes connus en soi, sont couramment utilisés dans la construction de certains instruments d'écriture à mine ou à bille rétractable.

Sur la figure 18A, on voit la position rétractée de la tige d'accouplement 9 dans le tube 8 du mandrin directeur 1, avant engagement de l'ergot d'accouplement 16b du mandrin d'appui 5, dans le passage transversal 14 ménagé dans l'extrémité distale 4a dudit mandrin directeur.

La figure 18B montre l'ergot d'accouplement 16b du mandrin d'appui 5 engagé dans le passage transversal 14 de l'extrémité distale du mandrin directeur 1, dans une position suivant laquelle le cran ou entaille transversale 16c se trouve placé dans l'alignement de la tige d'accouplement 9.

La figure 18C montre l'insertion de l'extrémité distale de la tige d'accouplement 9 dans le cran de la tête d'accouplement 16 du mandrin d'appui 5, obtenue par l'enfoncement du bouton-poussoir 25.

La figure 18D montre la fixation du mandrin d'appui 5 sur le mandrin directeur 1.

La séparation du mandrin d'appui et du mandrin directeur, à la fin de l'intervention chirurgicale, s'obtient par une simple pression sur le bouton-poussoir 25 lequel actionne le mécanisme assurant la rétraction de la tige d'accouplement 9.

D'autres moyens pourraient être mis en oeuvre pour assurer la jonction et la séparation rapides ou quasi instantanées du mandrin d'appui et du mandrin directeur, par exemple des moyens utilisant la force électromagnétique.

## Revendications

1. Ecarteur-rétenteur d'intestins pour chirurgie coelioscopique, qui comprend :
- un mandrin directeur (1) comportant une partie proximale (2) constituée par un manche (2) apte à être relié à une table d'opération, une partie distale (4) de forme allongée appelée à être introduite dans la cavité abdominale lors d'une intervention laparoscopique ;
- un mandrin d'appui (5); l'extrémité distale de ce mandrin d'appui (5) et la partie distale du mandrin directeur (1) étant pourvues de moyens complémentaires d'accouplement rapide (14, 9 ; 16b, 16c) ;
**caractérisé en ce que** :
- le mandrin d'appui (5) est de forme allongée sur lequel et le long duquel est fixée une nappe de contention, repliée selon un mode de pliage permettant son déploiement in situ , l'écarteur-rétenteur comportant un instrument (7) pour l'insertion du mandrin d'appui (5) et de la nappe de contention dans la cavité abdominale et sa fixation sur le mandrin directeur, cet instrument comportant un canon (23) dont au moins la partie distale (23c) est creuse et dimensionnée pour recevoir ledit mandrin d'appui (5) et la nappe de contention reliée à ce dernier ;
- le mandrin d'appui (5) est réalisé sous forme de gouttière (15) dans laquelle est logée la nappe de contention à l'état replié, ladite nappe étant fixée, par l'intermédiaire de l'un de ses bords (6D) dans ladite gouttière (15).

2. Ecarteur-rétenteur d'intestins, selon la revendication 1, dans lequel la nappe de contention est un filet de contention (6).

3. Ecarteur-rétenteur d'intestins selon la revendication 1 ou 2, **caractérisé en ce que** la partie distale (4) du mandrin directeur (1) est constituée par un tube (8) dans lequel est logée une tige d'accouplement (9) montée avec une aptitude de mouvement axial dans ledit tube (8) dont l'extrémité distale (4a) est munie d'un passage transversal (14), l'extrémité distale du mandrin d'appui (5) comportant un ergot (16b) destiné à s'insérer dans ledit passage transversal et muni d'un cran (16c) dans lequel peut être engagée l'extrémité distale de la tige d'accouplement (9), de sorte à fixer le mandrin d'appui (5) sur le mandrin directeur (1).

4. Ecarteur-rétenteur d'intestins suivant la revendication 3, **caractérisé en ce que** la tige d'accouplement (9) est soumise à l'action d'un ressort (12) tendant à la repousser en position d'accouplement, de sorte que la fixation du mandrin d'appui (5) sur le mandrin directeur (1) s'opère automatiquement par simple enfoncement de l'ergot d'accouplement dudit mandrin d'appui dans le passage transversal (14) dudit mandrin directeur.

5. Ecarteur-rétenteur d'intestins, selon la revendication 3, **caractérisé en ce que** le manche (2) du mandrin directeur (1) renferme un mécanisme à commande par bouton-poussoir (25) permettant d'obtenir, alternativement, par des pressions successives sur ce bouton-poussoir, soit l'introduction de l'extrémité de la tige d'accouplement (9) dans le passage transversal (14), soit la rétraction de ladite extrémité dans le tube (8).

6. Ecarteur-rétenteur d'intestins selon l'une quelconque des revendications 1 à 5, **caractérisé en ce que** le mandrin d'appui (5) est réalisé sous forme de gouttière (15) de section en arc de cercle.

7. Ecarteur-rétenteur d'intestins selon l'une quelconque des revendications 1 à 6, **caractérisé en ce que** la nappe de contention présente une forme constituée par:
une surface rectangulaire (6A), une première surface triangulaire (6B) constituée par un triangle rectangle rattaché par son grand côté de l'angle droit à l'un des petits côtés de la surface rectangulaire (6A), et une deuxième surface triangulaire (6C) constituée par un triangle rectangle rattaché par son grand côté de l'angle droit à l'un des grands côtés de la surface rectangulaire (6A), et **en ce que** le petit côté de l'angle droit du premier triangle (6B) se trouve disposé dans le prolongement de l'un des grands côtés du rectangle (6A), tandis que le petit côté de l'angle droit du deuxième triangle (6C) se trouve disposé dans le prolongement du petit côté du rectangle (6A) qui forme un angle droit avec le grand côté dudit rectangle prolongé par le petit côté de l'angle droit dudit premier triangle (6B).

8. Ecarteur-rétenteur d'intestins selon la revendication 7, **caractérisé en ce que** la nappe de contention est fixée au mandrin d'appui (5) par l'intermédiaire de sa bordure (6D) délimitée par les sommets (S1 et S2) des surfaces triangulaires (6B, 6C), constitués par les angles formés par leur hypoténuse et le grand côté de leur angle droit.

9. Ecarteur-rétenteur d'intestins suivant l'une quelconque des revendications 1 à 7, **caractérisé en ce que** le bord libre de la nappe de contention est renforcé.

10. Ecarteur-rétenteur d'intestins selon la revendication 9, dans lequel la nappe présente un degré d'élasticité, et dans lequel le bord libre de la nappe de contention est renforcé par une bordure (18), de préférence constituée par des revers cousus par des fils non élastiques (19).

11. Ecarteur-rétenteur d'intestins suivant l'une quelconque des revendications 1 à 10, **caractérisé en ce que** les angles ou sommets (S3, S4, S5) de la nappe de contention destinés à être attachés à la paroi abdominale du patient pour l'exécution d'une intervention laparoscopique sont munis d'aiguilles (20a, 20b, 20c) reliées par des fils (21) auxdits angles ou sommets.

12. Ecarteur-rétenteur d'intestins selon la revendication 11, **caractérisé en ce que** les angles ou sommets (S3, S4, S5) de la nappe de contention sont munis de petits fourreaux (22) dans lesquels sont logées les aiguilles (20a, 20b, 20c).

13. Ecarteur-rétenteur d'intestins selon l'une quelconque des revendications 1 à 12, **caractérisé en ce que** la nappe de contention est pliée en accordéon.

14. Ecarteur-rétenteur d'intestins selon l'une quelconque des revendications 1 à 12, dans lequel la nappe de contention est enroulée.

15. Ecarteur-rétenteur d'intestins selon l'une quelconque des revendications 1 à 5, **caractérisé en ce que** l'extrémité distale du mandrin d'appui (5) est constituée par une tête d'accouplement (16) dont la face postérieure (16a) remplit la fonction d'arrêtoir placé en butée contre l'extrémité distale du canon (23) de l'instrument d'insertion (7).

16. Ecarteur-rétenteur d'intestins selon la revendication 15, **caractérisé en ce que** la face avant de la tête d'accouplement (16) comporte une cavité diamétrale (16d) de forme incurvée, cette cavité étant conformée pour épouser la forme cylindrique de la paroi latérale de la partie distale (4) du mandrin directeur (1).

17. Ecarteur-rétenteur d'intestins selon l'une quelconque des revendications 15 ou 16, **caractérisé en ce que** la tête d'accouplement (16) est munie d'un ergot de clipsage (16b) de forme tronconique, et **en ce que** l'extrémité distale de la tige mobile d'accouplement (9) du mandrin directeur (1) émerge dans un passage transversal (14) ménagé dans la partie distale dudit mandrin directeur (1) et présente un biseau (9a) contre lequel glisse ledit ergot lors de son introduction dans ledit passage, en entraînant le soulèvement de ladite tige d'accouplement.

18. Ecarteur-rétenteur d'intestins suivant l'une quelconque des revendications 1 à 4, 16 ou 17, **caractérisé en ce que** l'extrémité proximale de la tige d'accouplement (9) est solidaire d'un bouton de manoeuvre (10) accessible à travers une ouverture (11) ménagée latéralement dans le manche (2) du mandrin directeur (1), un ressort (12) prenant appui sur la face supérieure dudit bouton (10) de sorte à repousser la tige d'accouplement (9) en position d'accouplement.

## Patentansprüche

1. Spreizer-Zurückhalter des Darms zur zöliskopischen Chirurgie, der umfasst:
- eine Leitspindel (1), umfassend einen proximalen Teil (2), der durch einen Griff (2) gebildet wird, der geeignet ist, mit einem Operationstisch verbunden zu werden, einen distalen Teil (4) in verlängerter Form, der dazu bestimmt ist, bei einem laparoskopischen chirurgischen Eingriff in die Bauchhöhle eingefügt zu werden;
- eine Stützspindel (5); wobei das distale Ende dieser Stützspindel (5) und der distale Teil der Leitspindel (1) mit komplementären Mitteln zur schnellen Kopplung (14, 9; 16b, 16c) versehen sind;
**dadurch gekennzeichnet, dass**:
- die Stützspindel (5) eine längliche Form aufweist, auf der und entlang der ein Kompressionstuch befestigt ist, das gemäß einem Faltmodus umgeschlagen ist, der sein Entfalten in situ erlaubt, wobei der Spreizer-Zurückhalter ein Instrument (7) zum Einführen der Stützspindel (5) und des Kompressionstuchs in der Bauchhöhle und dessen Befestigung auf der Leitspindel umfasst, wobei dieses Instrument ein Rohr (23) umfasst, von dem wenigstens der distale Teil (23c) hohl und ausgelegt ist, um die genannte Stützspindel (5) und das mit diesem verbundene Kompressionstuch aufzunehmen;
- die Stützspindel (5) in Form einer Rinne (15) realisiert ist, in der das Kompressionstuch im umgeschlagenen Zustand untergebracht ist, wobei das genannte Tuch über eines seiner Ränder (6D) in der genannten Rinne (15) befestigt ist.

2. Spreizer-Zurückhalter des Darms gemäß Anspruch 1, in dem das Kompressionstuch ein Kompressionsnetz (6) ist.

3. Spreizer-Zurückhalter des Darms gemäß Anspruch 1 oder 2, **dadurch gekennzeichnet, dass** der distale Teil (4) der Leitspindel (1) durch eine Röhre (8) gebildet ist, in der ein Kopplungsstift (9) untergebracht ist, der in einer axialen Bewegungsfähigkeit in der genannten Röhre (8) montiert ist, dessen distales Ende (4a) mit einem transversalen Durchgang (14) versehen ist, wobei das distale Ende der Stützspindel (5) eine Nocke (16b) umfasst, die dazu bestimmt ist, sich in den genannten transversalen Durchgang einzufügen und mit einem Einschnitt (16c) versehen ist, in den das distale Ende des Kopplungsstiftes (9) derart eingefügt werden kann, dass die Stützspindel (5) auf der Leitspindel (1) befestigt wird.

4. Spreizer-Zurückhalter des Darms gemäß Anspruch 3, **dadurch gekennzeichnet, dass** der Kopplungsstift (9) der Wirkung einer Feder (12) unterworfen ist, die dazu neigt, ihn in die Kopplungsposition derart zurückzuschieben, dass die Befestigung der Stützspindel (5) auf der Leitspindel (1) automatisch per einfachem Eindrücken der Kopplungsnocke der genannten Stützspindel in den transversalen Durchgang (14) der genannten Leitspindel erfolgt.

5. Spreizer-Zurückhalter des Darms gemäß Anspruch 3, **dadurch gekennzeichnet, dass** der Griff (2) der Leitspindel (1) einen Steuermechanismus per Drucktaster (25) einschließt, der durch sukzessive Drücke auf diesen Drucktaster alternativ entweder das Einführen des Endes des Kopplungsstiftes (9) in den transversalen Durchgang (14) oder das Zurückziehen des genannten Endes in der Röhre (8) erlaubt.

6. Spreizer-Zurückhalter des Darms gemäß Anspruch 1 bis 5, **dadurch gekennzeichnet, dass** die Stützspindel (5) in Form einer Rinne (15) mit einem Kreisbogen-Querschnitt realisiert ist.

7. Spreizer-Zurückhalter des Darms gemäß Anspruch 1 bis 6, **dadurch gekennzeichnet, dass** das Kompressionstuch eine Form aufweist, die gebildet wird durch: eine rechteckige Fläche (6A), eine erste dreieckige Fläche (6B), die durch ein rechtwinkliges Dreieck gebildet wird, das an seiner langen Seite des rechten Winkels an einer der kleinen Seiten der rechteckigen Fläche (6A) befestigt ist, und eine zweite dreieckige Fläche (6C), die durch ein rechtwinkliges Dreieck gebildet wird, das an seiner langen Seite des rechten Winkels an einer der langen Seiten der rechteckigen Fläche (6A) befestigt ist, und dass die kurze Seite des rechten Winkels des ersten Dreiecks (6B) sich in der Verlängerung einer der langen Seiten des Rechtecks (6A) angeordnet befindet, während die kurze Seite des rechten Winkels des zweiten Dreiecks (6C) sich in der Verlängerung der kurzen Seiten des Rechtecks (6A) angeordnet befindet, das einen rechten Winkel mit der langen Seite des genannten Rechtecks bildet, die durch die kurze Seite des rechten Winkels des genannten ersten Dreiecks (6B) verlängert wird.

8. Spreizer-Zurückhalter des Darms gemäß Anspruch 7, **dadurch gekennzeichnet, dass** das Kompressionstuch an der Stützspindel (5) durch seine Umrandung (6D) befestogt ist, die durch die Scheitelpunkte (S1 und S2) der dreieckigen Flächen (6B, 6C) begrenzt wird, welche durch Winkel gebildet werden, die durch ihre Hypotenuse und die lange Seite ihres rechten Winkels gebildet sind.

9. Spreizer-Zurückhalter des Darms gemäß Anspruch 1 bis 7, **dadurch gekennzeichnet, dass** der freie Rand des Kompressionstuchs verstärkt ist.

10. Spreizer-Zurückhalter des Darms gemäß Anspruch 9, bei dem das Tuch einen Elastizitätsgrad aufweist und bei dem der freie Rand des Kompressionstuchs durch eine Umrandung (18) verstärkt ist, die bevorzugt durch Umschläge gebildet ist, die mit nicht elastischen Fäden (19) genäht sind.

11. Spreizer-Zurückhalter gemäß Anspruch 1 bis 10, **dadurch gekennzeichnet, dass** die Winkel oder Scheitelpunkte (S3, S4, S5) des Kompressionstuchs, die dazu bestimmt sind, an der Bauchdecke des Patienten zur Durchführung eines laparoskopischen chirurgischen Eingriffs befestigt zu werden, mit Nadeln (20a, 20b, 20c) versehen sind, die durch Fäden (21) an den genannten Winkeln oder Scheitelpunkten verbunden sind.

12. Spreizer-Zurückhalter des Darms gemäß Anspruch 11, **dadurch gekennzeichnet, dass** die Winkel oder Scheitelpunkte (S3, S4, S5) des Kompressionstuchs mit kleinen Hülsen (22) versehen sind, in denen die Nadeln (20a, 20b, 20c) untergebracht sind.

13. Spreizer-Zurückhalter des Darms gemäß Anspruch 1 bis 12, **dadurch gekennzeichnet, dass** das Kompressionstuchs wie ein Akkordeon gefaltet ist.

14. Spreizer-Zurückhalter des Darms gemäß Anspruch 1 bis 12, bei dem das genannte Kompressionstuch eingerollt ist.

15. Spreizer-Zurückhalter des Darms gemäß Anspruch 1 bis 5, **dadurch gekennzeichnet, dass** das distale Ende der Stützspule (5) durch einen Kopplungskopf (16) gebildet ist, dessen hintere Seite (16a) die Arretierfunktion erfüllt, die im Anschlag gegen das distale Ende des Rohrs (23) des Einführinstruments (7) platziert ist.

16. Spreizer-Zurückhalter des Darms gemäß Anspruch 15, **dadurch gekennzeichnet, dass** die vordere Seite des Kopplungskopfes (16) eine diametrale Höhle (16d) in gekrümmter Form aufweist, wobei diese Höhle angepasst ist, um die zylindrische Form der lateralen Wand des distalen Teils (4) der Leitspindel (1) zu übernehmen.

17. Spreizer-Zurückhalter des Darms gemäß Anspruch 15 oder 16, **dadurch gekennzeichnet, dass** der Kopplungskopf (16) mit einer Verklippungsnocke (16b) in kegelstumpfartiger Form versehen ist und dass das distale Ende des mobilen Kopplungsstiftes (9) der Leitspindel (1) in einem transversalen Durchgang (14) der in dem distalen Teil der genannten Leitspindel (1) ausgespart ist, hervorsteht und eine Abschrägung (9a) aufweist, gegen die die genannte Nocke bei ihrem Einführen in den genannten Durchgang gleitet, indem sie die Erhebung des genannten Kopplungsstiftes nach sich zieht.

18. Spreizer-Zurückhalter des Darms gemäß Anspruch 1 bis 4, 16 oder 17, **dadurch gekennzeichnet, dass** das proximale Ende des Kopplungsstiftes (9) fest mit einem Steuerknopf (10) verbunden ist, der über eine Öffnung (11) zugänglich ist, die lateral in dem Griff (2) der Leitspindel (1) ausgespart ist, wobei eine Feder (12) sich auf der oberen Seite des genannten Knopfes (10) derart aufstützt, dass der Kopplungsstift (9) in Kopplungsposition zurückgeschoben wird.

## Claims

1. A retractor-retainer of intestines for celioscopic surgery, which comprises:
- a guiding mandrel (1) having a proximal portion (2) consisting of a handle (2) adapted to be connected to a operating table, a distal portion (4) having an elongated shape designed to be introduced into the abdominal cavity during a laparoscopic operation;
- a support mandrel (5); the distal part of same support mandrel (5) and the distal part of the guiding mandrel (1) being provided with complementary quick coupling means (14, 9; 16b, 16c),
**characterized in that**
- the support mandrel (5) has an elongated shape on and along which a retaining web is fixed, folded according to a folding method enabling the deployment in situ thereof, the retractor-retainer comprising an instrument (7) for inserting the support mandrel (5) and the retaining web into the abdominal cavity and the attachment thereof to the guiding mandrel, such instrument comprising a barrel (23) the distal portion (23c) of which at least is hollow and sized so as to receive said support mandrel (5) and the retaining web connected thereto;
- the support mandrel (5) has the shape of a splint (15), wherein the retaining web is housed, in a folded condition, with said web being attached by one of the edges (6D) thereof in said splint (15).

2. A retractor-retainer of intestines according to claim 1, wherein the retaining web is a retaining net (6).

3. A retractor-retainer of intestines according to claim 1 or 2, **characterized in that** the distal portion (4) of the guiding mandrel (1) comprises a tube (8) wherein a coupling rod (9) is accommodated and mounted with an axial mobility allowance in said tube (8) the distal end (4a) of which is provided with a transverse passage (14), the distal end of the support mandrel (5) having a pin (16b) intended to be inserted into said transverse passage and provided with a notch (16c) wherein the distal end of the coupling rod (9) can be engaged so as to fix the support mandrel (5) on the guiding mandrel (1).

4. A retractor-retainer of intestines according to claim 3, **characterized in that** the coupling rod (9) is subjected to the action of a spring (12) tending to push back to the coupling position, so that the attachment of the support mandrel (5) to the guiding mandrel (1) is automatically performed simply by depressing the coupling pin of said support mandrel into the transverse passage (14) of said guiding mandrel.

5. A retractor-retainer of intestines according to claim 3, **characterized in that** the handle (2) of the guiding mandrel (1) contains a push-button (25) controlled mechanism making it possible to obtain, alternately, by successively pressing the push-button, either the introduction of the end of the coupling rod (9) into the transverse passage (14) or the retraction of said end into the tube (8).

6. A retractor-retainer of intestines according to any one of claims 1 to 5, **characterized in that** the support mandrel (5) is designed as a splint (15) having a semi-circular section.

7. A retractor-retainer of intestines according to any one of claims 1 to 6, **characterized in that** the retaining web shaped by : a rectangular surface (6A), a first triangular face (6B) constituting a right-angled triangle connected by the longer side of the right angle to one of the shorter sides of the rectangular surface (6A), and a second triangular face (6C) constituting a right-angled triangle connected by the longer side of the right angle to one of the longer sides of the rectangular surface (6A), and **in that** the shorter side of the right angle of the first triangle (6B) is so arranged as to be running on from one of the longer sides of the rectangle (6A), whereas the shorter side of the right angle of the second triangle (6C) is so arranged as to be running on from the shorter side of the rectangle (6A) which forms a right angle with the longer side of said rectangle extended by the shorter side of the right angle of said first triangle (6B).

8. A retractor-retainer of intestines according to claim 7, **characterized in that** the retaining web is attached to the support mandrel (5) via the border (6D) thereof delimited by the vertices (SI and S2) of the triangular surfaces (6B, 6C), formed by the angles formed by the hypotenuses thereof and the longer side of the right angle thereof.

9. A retractor-retainer of intestines according to any one of Claims 1 to 7, **characterized in that** the free edge of the retaining web is reinforced.

10. A retractor-retainer of intestines according to claim 9, wherein the web has a degree of elasticity, and wherein the free edge of the retaining web is reinforced by a border (18), preferably constituted by backs sewn with non-elastic thread (19).

11. A retractor-retainer of intestines according to any one of Claims 1 to 10, **characterized in that** the angles or vertices (S3, S4, S5) of the retaining web intended to be attached to the patient's abdominal wall for the celioscopic surgery is provided with needles (20a, 20b, 20c) connected by threads (21) to said angles or vertices.

12. A retractor-retainer of intestines according to claim 11, **characterized in that** the angles or vertices (S3, S4, S5) of the retaining web are provided with small sheathes (22) wherein the needles (20a, 20b, 20c) are accommodated.

13. A retractor-retainer of intestines according to any one of claims 1 to 12, **characterized in that** the retaining web is fanfold.

14. A retractor-retainer of intestines according to any one of claims 1 to 12, wherein the retaining web is wound.

15. A retractor-retainer of intestines according to any one of claims 1 to 5, **characterized in that** the distal end of the support mandrel (5) consists of a coupling head (16) the back face (16a) of which serves as a stopper placed in abutment against the distal end of the barrel (23) of the insertion instrument (7).

16. A retractor-retainer of intestines according to claim 15, **characterized in that** the front face of the coupling head (16) comprises a diametral recess (16d) having a curved shape, with said cavity being shaped to match the cylindrical shape of the side wall of the distal portion (4) of the guiding mandrel (1).

17. A retractor-retainer of intestines according to one of claims 15 or 16, **characterized in that** the coupling head (16) is provided with a clipping pin (16b) having a truncated shape, and **in that** the distal end of the movable coupling rod (9) of the guiding mandrel (1) emerges in a transverse passage (14) provided in the distal portion of said guiding mandrel (1) and has a bevel (9a) against which said pin slides upon the introduction thereof into said passage, resulting in the lifting of said coupling rod.

18. A retractor-retainer of intestines according to any one of claims 1 to 4, 16 or 17, **characterized in that** the proximal end of the coupling rod (9) is secured to an actuating button (10) accessible through an opening (11) provided laterally in the handle (2) of the guiding mandrel (1), a spring (12) resting on the upper face of said button (10) so as to push the connecting rod (9) back to the coupling position.
